# EUROPÄISCHE PATENTANMELDUNG

(11) **EP 1 101 508 A2**
(43) Veröffentlichungstag der Anmeldung: **23.05.2001**
(21) Anmeldenummer: 00119216.0
(22) Anmeldetag: 06.09.2000
(51) Int. Cl.: A61M 25/06

(54) **Vorrichtung zum Einführen von Gegenständen in die Blütbahn eines Patienten**

(30) Priorität: 18.11.1999 DE 19955445
(71) Anmelder: Joline GmbH & Co. KG, 72379 Hechingen (DE)
(72) Erfinder: Sunnanväder, Lars, 72379 Hechingen (DE); Hartig, Tomas, 72379 Hechingen (DE)
(74) Vertreter: Möbus, Daniela, Dr.-Ing.

(57) **Zusammenfassung**

Vorrichtung (12) zum Einführen von Gegenständen in die Blutbahn eines Patienten über einen Katheter (1.0) mit einem Dilator (11), die ein am hinteren Ende des Katheters (10) anordenbares hämostatisches Ventil (12) ist, wobei das hämostatische Ventil (12) als ein Rückschlagventil ausgebildet ist und den Dilator (11) durch mindestens eine umlaufende Dichtung im Ventil (12) umfasst.

## Beschreibung

Die Erfindung betrifft eine Vorrichtung zum Einführen von Gegenständen in die Blutbahn eines Patienten über einen Katheter mit einem Dilator.

Um Gegenstände, insbesondere Katheter und Kreislaufunterstützungssysteme wie z. B. eine Herzpumpe, in den Körper eines Patienten einzuführen, wird seither zunächst ein Katheter in eine Vene oder die Aorta eingeführt, über dessen offenes Ende anschließend der einzuführende Gegenstand eingeführt wird. Sobald jedoch der Katheter in die Vene oder die Aorta eingeführt ist und der zugehörige eingeschobene Dilator herausgezogen wird, strömt Blut in den Katheter ein, sodass es zwangsläufig vor der Einführung des zu positionierenden Gegenstandes durch das offene Ende des Katheters zu einem unerwünschten Blutverlust kommt.

Die Erfindung hat die Aufgabe, eine Vorrichtung zum Einführen von Gegenständen in die Blutbahn vorzuschlagen, mit der zukünftig beim Einbringen des einzuführenden Gegenstandes keine nennenswerten Mengen an Blut mehr ausströmen.

Die Erfindung löst die gestellte Aufgabe durch eine eingangs genannte Vorrichtung, die ein am hinteren Ende des Katheters anordenbares hämostatisches Ventil ist, wobei das hämostatische Ventil als ein Rückschlagventil ausgebildet ist und den Dilator durch mindestens eine umlaufende Dichtung im Ventil umfasst. Durch die mindestens eine den Dilator umfassende Dichtung kann beim Herausziehen des eingeschobenen Dilators am oben offenen Ende des hämostatischen Ventils so gut wie kein Blut in die Umgebung austreten. Dadurch wird der Blutverlust erheblich reduziert. Nachdem der Dilator vollständig aus dem Ventil herausgezogen ist, schließt das Rückschlagventil den Katheter zur Umgebung hin dicht ab. Das entsprechende Abdichten erfolgt beim Entfernen des Katheters. Somit kann zu keinem Zeitpunkt Blut nach außen dringen.

Der Körper des hämostatischen Ventils kann aus zwei in Längsrichtung zusammensetzbaren Hälften bestehen. Wenn der eingeführte Gegenstand in der gewünschten Position im Körper des Patienten liegt, werden der Katheter samt Dilator und das hämostatische Ventil nicht mehr benötigt. Um das Ventil dann entfernen zu können, kann es in seine beiden in Längsrichtung zusammensetzbaren Hälften zerlegt und abgenommen werden.

Damit die beiden Hälften des Körpers des hämostatischen Ventils schnell und einfach voneinander getrennt werden können, können die beiden Hälften des Ventils durch auftrennbare Befestigungsmittel zusammengehalten sein. Solche auftrennbare Befestigungsmittel können beispielsweise Gummibänder, O-Ringe, Schnüre, Kabelbinder oder dergleichen sein. Genauso vorteilhaft ist es auch, wenn die beiden Hälften des Körpers an seinem Ende durch eine überwurfmutter zusammengehalten werden, die nachdem das Ventil nicht mehr benötigt wird, von ihm heruntergeschraubt werden kann.

Das Rückschlagventil kann von zwei im geschlossenen Zustand des Ventils aneinander anliegenden elastischen Dichtlippen gebildet sein. Diese Dichtlippen liegen vorteilhafterweise genau in der Trennebene der beiden in Längsrichtung zusammensetzbaren Hälften des hämostatischen Ventils aneinander an.

Aus Gründen einer einfachen Montage und Demontage des hämostatischen Ventils kann das Rückschlagventil und die mindestens eine umlaufende Dichtung als ein Teil hergestellt sein, wobei dieses eine Teil aus zwei in Längsrichtung durch Einschneiden oder dergleichen hergestellten Hälften bestehen kann.

Damit ein anzuschließender Schlauch einer Herzpumpe oder dergleichen ohne großen Blutverlust in den Katheter eingeschoben werden kann, kann das hämostatische Ventil mindestens eine umlaufende Dichtung für einen anzuschließenden Schlauch oder dergleichen aufweisen.

Wenn der Körper des hämostatischen Ventils eine Rastverbindung zum Anschließen des Katheters aufweist, so können mit einem einfachen Handgriff schnell Ventil und Katheter miteinander verbunden werden.

Alternativ dazu kann zum einfachen Anschließen des Ventils mit dem Katheter der Körper des hämostatischen Ventils durch eine Mutter mit dem Katheter verbunden werden.

Vorteilhafterweise kann der Körper des hämostatischen Ventils zusätzlich einen seitlichen Anschluss für die Zugabe von Medikamenten aufweisen.

Nachfolgend wird ein Ausführungsbeispiel einer erfindungsgemäßen Vorrichtung anhand der beiliegenden Zeichnung näher erläutert.

Im Einzelnen zeigen:
- Fig. 1: eine Prinzipskizze einer erfindungsgemäßen Vorrichtung zusammen mit einem Katheter und Dilator;
- Fig. 2: einen Längsschnitt durch das hämostatische Ventil aus Fig. 1.

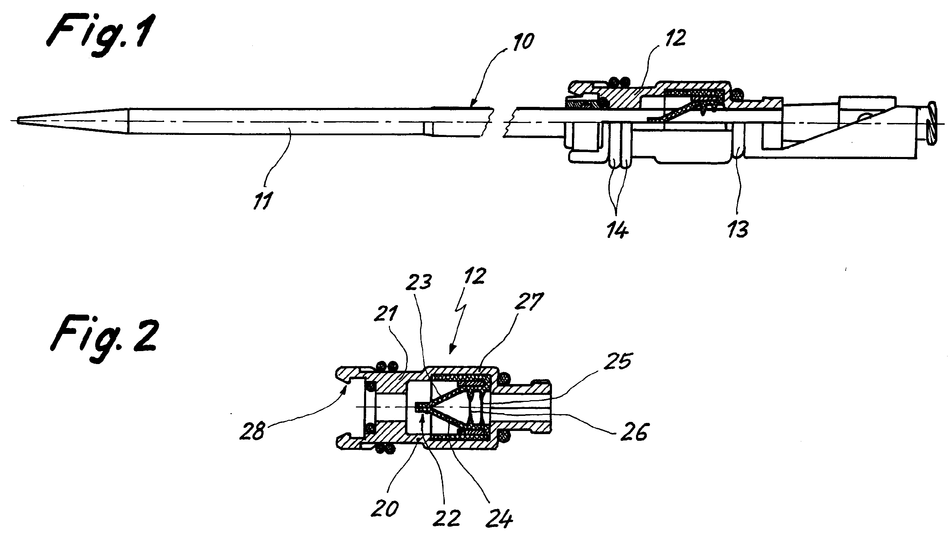

Fig. 1 zeigt einen Katheter 10 mit einem hämostatischen Ventil 12, in den ein Dilator 11 eingeschoben ist. Zunächst wird der Dilator 11 zusammen mit dem Katheter 10 in eine Vene oder die Aorta eingeführt. Anschließend wird der das Eindringen des Blutes in den Katheter 10 verhindernde Dilator 11 nach hinten aus dem Katheter 10 herausgezogen. Sobald der Dilator 11 auch aus dem auf dem hinteren Ende des Katheters 10 aufgesetzten hämostatischen Ventil 12 herausgezogen ist, verhindert das hämostatische Ventil 12 ein Austreten des Blutes aus dem Katheter 10 in die Umgebung. Als Nächstes wird ein in den Körper des Patienten einzuführender Gegenstand in die hintere öffnung des hämostatischen Ventils 12 eingeschoben, bis dieser in der gewünschten Position im Körper des Patienten liegt. Nach dem Einschieben des Gegenstandes durch den Katheter 10 und eines verbundenen Gegenstandes, beispielsweise einem Schlauch 13 oder einer Herzpumpe, werden das hämostatische Ventil 12 und der Katheter 10 samt Dilator 11 nicht mehr benötigt. Da das hämostatische Ventil 12 aus zwei in Längsrichtung zusammensetzbaren Hälften 20 und 21 (Fig. 2) besteht, können diese nach dem Durchtrennen von die Hälften 20 und 21 zusammenhaltenden 0-Ringen 13 und 14 vom Schlauch entfernt werden. Dann wird auch der teilbare Katheter 10 herausgezogen, geteilt und entfernt.

Fig. 2 zeigt das hämostatische Ventil 12 aus Fig. 1 im Detail. Dieses besteht aus zwei in Längsrichtung zusammensetzbaren Hälften 20 und 21. Das hämostatische Ventil 12 beinhaltet ein Rückschlagventil 22, das seinerseits ebenfalls aus zwei in Längsrichtung zusammensetzbaren Hälften 23 und 24 besteht. Im geschlossenen Zustand des Rückschlagventils 22 liegen die beiden jeweils eine elastische Dichtlippe beinhaltenden Hälften 23 und 24 aneinander an. Außerdem beinhaltet das hämostatische Ventil 12 zwei umlaufende Dichtungen 25 und 26, die beim Einschieben des Dilators 11 in das hämostatische Ventil 12 und bei seiner Entfernung bzw. beim Einschieben eines nicht näher dargestellten Gegenstandes ein Austreten von Blut in die Umgebung verhindern. Auch die umlaufenden Dichtungen 25 und 26 können jeweils als zwei in Längsrichtung zusammensetzbare Hälften ausgebildet sein. Damit das Rückschlagventil 22 und die beiden umlaufenden Dichtungen 25 und 26 leicht in die Hälften des Gehäuses 27 des hämostatischen Ventils 12 eingebaut werden können, können ihre Hälften vorteilhafterweise als ein Teil gefertigt sein, welches durch Schneiden in zwei Teile getrennt werden kann. Im vorderen Bereich weist das hämostatische Ventil 12 einen Rastvorsprung 28 auf, in den der Katheter 10 einschnappen kann.

## Patentansprüche

1. Vorrichtung (12) zum Einführen von Gegenständen in die Blutbahn eines Patienten über einen Katheter (10) mit einem Dilator (12), dadurch gekennzeichnet, dass sie ein am hinteren Ende des Katheters (10) anordenbares hämostatisches Ventil (12) ist, wobei das hämostatische Ventil (12) als ein Rückschlagventil (22) ausgebildet ist und den Dilator (11) durch mindestens eine umlaufende Dichtung (25, 26) im Ventil (12) umfasst.

2. Vorrichtung (12) nach Anspruch 1, dadurch gekennzeichnet, dass der Körper (27) des hämostatischen Ventils (12) aus zwei in Längsrichtung zusammensetzbaren Hälften (20, 21) besteht.

3. Vorrichtung (12) nach Anspruch 2, dadurch gekennzeichnet, dass die beiden Hälften (20, 21) des Körpers (27) des hämostatischen Ventils (12) durch auftrennbare Befestigungsmittel (13, 14) zusammengehalten werden.

4. Vorrichtung (12) nach Anspruch 2, dadurch gekennzeichnet, dass die beiden Hälften (20, 21) des Körpers (27) des hämostatischen Ventils (12) durch Muttern zusammengehalten werden.

5. Vorrichtung (12) nach einem der Ansprüche 1 bis 4, dadurch gekennzeichnet, dass das Rückschlagventil (22) von zwei zusammensetzbaren, im geschlossenen Zustand des Ventils aneinander anliegenden elastischen Dichtlippen (23, 24) gebildet ist.

6. Vorrichtung (12) nach einem der Ansprüche 1 bis 5, dadurch gekennzeichnet, dass das Rückschlagventil (22) und die mindestens eine umlaufende Dichtung (25, 26) als ein Teil hergestellt sind, wobei dieses eine Teil aus zwei in Längsrichtung zusammensetzbaren Hälften (23, 24) besteht.

7. Vorrichtung (12) nach einem der Ansprüche 1 bis 6, dadurch gekennzeichnet, dass das hämostatische Ventil (12) mindestens eine umlaufende Dichtung (25, 26) für einen anzuschließenden Schlauch oder dergleichen aufweist.

8. Vorrichtung (12) nach einem der Ansprüche 1 bis 7, dadurch gekennzeichnet, dass der Körper (27) des hämostatischen Ventils (12) eine Rastverbindung (28) zum Anschließen an den Katheter aufweist.

9. Vorrichtung (12) nach einem der Ansprüche 1 bis 7, dadurch gekennzeichnet, dass der Körper (27) des hämostatischen Ventils (12) durch eine Mutter mit dem Katheter (10) verbindbar ist.

10. Vorrichtung (12) nach einem der Ansprüche 1 bis 9, dadurch gekennzeichnet, dass der Körper (27) des hämostatischen Ventils (12) seitlich einen Anschluss für die Zugabe von Medikamenten oder dergleichen aufweist.
